# FASCICULE DE BREVET EUROPEEN

(11) **EP 3 413 849 B1**
(45) Date de publication et mention de la délivrance du brevet: **29.01.2020**
(21) Numéro de dépôt: 17706169.4
(22) Date de dépôt: 06.02.2017
(51) Int. Cl.: A61F 5/08

(54) **ENSEMBLE D'INSTRUMENTS DE MODELAGE DU NEZ, PROCÉDÉ DE RÉALISATION ET PROCÉDÉ DE MODELAGE DU NEZ**
SET VON NASENMODELLIERINSTRUMENTEN, HERSTELLUNGSVERFAHREN UND VERFAHREN ZUR MODELLIERUNG DER NASE
SET OF NOSE-MODELLING INSTRUMENTS, PRODUCTION METHOD AND A METHOD FOR MODELLING THE NOSE

(30) Priorité: 09.02.2016 FR 1651022
(43) Date de publication de la demande: 19.12.2018
(73) Titulaire: S.e.l.a.r.l De Medecins Plasticiens Paris, 75116 Paris (FR)
(72) Inventeur: BENJOAR, David Marc, 75116 Paris (FR); BERDAH, Yaël, 75116 Paris (FR)
(74) Mandataire: IPAZ
(86) Numéro de dépôt international: PCT/EP2017/052551
(87) Numéro de publication internationale: WO 2017/137356

(56) Documents cités:
- EP-A1- 1 908 438
- WO-A1-2011/148309
- CH-A1- 709 544
- US-A- 3 742 943

## Description

La présente invention a trait au domaine esthétique et plus particulièrement l'esthétique du nez d'un individu.

La présente invention concerne un procédé de modelage qui consiste à appliquer sur le nez de forme externe initiale, au moins deux instrument de modelage afin de guider la croissance cartilagineuse voire osseuse du nez et ainsi d'obtenir une forme externe finale visée du nez à la fin de la croissance de l'individu et qui est différente d'une forme externe naturelle qui serait obtenue naturellement en l'absence d'intervention.

L'invention concerne en outre un ensemble d'instruments de modelage dont les formes successives sont déterminées pour représenter une succession évolutive de une ou plusieurs formes intermédiaires visées depuis la forme externe initiale du nez et jusqu'à la forme externe finale visée.

Elle concerne aussi un procédé de réalisation des instruments de modelage au cours duquel, à partir de la forme externe initiale du nez, on détermine une forme finale visée et au moins une forme intermédiaire visée. On fabrique les instruments correspondant à chaque forme visée.

### Etat de la technique antérieure

Dans le domaine de la chirurgie esthétique, il est connu de modifier la forme du nez d'un individu dans un but d'amélioration esthétique. La méthode classique, l'intervention chirurgicale appelée rhinoplastie, vise à re-modeler le nez pour changer sa forme extérieure. Le nez est composé de plusieurs parties ou unités qui peuvent être modifiées séparément en diminuant certaines et augmentant d'autres. Le but de la rhinoplastie esthétique est d'obtenir une meilleure impression d'harmonie visuelle entre ces différentes parties, selon une forme qui aboutira à un nez d'aspect naturel et qui n'ait pas l'air d'être « refait ». Afin d'améliorer le processus de guérison suite à une opération de rhinoplastie, on connaît du document US 3 742 943 A une pluralité d'attelles à poser sur le nez en cours de dégonflement.

En général, il s'agit de corriger spécifiquement les disgrâces présentes, apparues notamment à l'adolescence. Typiquement, les rhinoplasties ont pour but d'amoindrir une bosse présente près du dos du nez (ou « dorsum ») et visible de profil.

Cette intervention n'est réalisable qu'à la fin de la croissance de l'individu, c'est-à-dire à partir d'environ 16 ans chez la femme ou environ 19 ans chez l'homme.

Les rhinoplasties ont lieu sous anesthésie générale ce qui rebute voire effraie une partie des sujets. Le coût de cette intervention est important puisqu'il nécessite plusieurs milliers d'euros.

Pour éviter ces inconvénients, il a été proposé d'intervenir sur la forme externe du nez en agissant lors de la croissance du visage en appliquant sur ce dernier un ou plusieurs dispositifs de contention localisé appelés « contenseurs nasaux », qui appliquent un effort en un ou plusieurs points spécifiques, en général en traction et parfois en appui. Ces contenseurs nasaux sont rigides et non déformables. Lorsque la croissance du nez rend douloureuse le port du contenseur, un nouveau dispositif de taille légèrement supérieure est mise en place.

On connaît du document WO 2011/148309 A1 un dispositif orthopédique pour le conditionnement de la croissance de la pyramide nasale pendant la période de croissance d'un individu. Un fil ressort est positionné sur la pyramide nasale afin d'exercer une pression sur quelques points de celle-ci. Cependant, les fils peuvent se déformer au cours du temps, ce qui rend le réglage très difficile voire approximatif et ne permet pas un suivi ni un pilotage précis de la croissance de la pyramide du nez de l'individu. Cette méthode nécessite d'ajuster régulièrement la forme du fil, en fonction de la pression que l'on souhaite exercer, et d'ajuster celle-ci au fur et à mesure pour se rapprocher de la forme visée.

Cette méthode n'a d'ailleurs pas été véritablement adoptée par les professionnels.

Le but de la présente invention est de pallier au moins un de ces inconvénients. L'invention cherche aussi à améliorer la prédictibilité des résultats, et à diminuer les inconforts encourus lors de modifications esthétiques de la forme du nez.

### Exposé de l'invention

L'invention est définie dans les revendications attenantes. Selon un premier aspect de l'invention, il est prévu un procédé de réalisation d'instruments de modelage de la forme du nez d'un individu. Le procédé de réalisation comprend les étapes suivantes :
- à partir d'une forme initiale, représentant la forme initiale d'une surface extérieure d'une région du nez dudit individu, dite région à modeler, telle qu'elle existe à un instant initial :
   ∘ détermination d'une forme finale visée, représentant la forme que l'on cherche à obtenir en un instant final, pour ladite région à modeler du nez dudit individu, et
   ∘ détermination d'au moins une forme intermédiaire visée, représentant une forme que l'on cherche à obtenir en un instant intermédiaire situé entre l'instant initial et l'instant final, pour ladite région à modeler du nez dudit individu ; et
- fabrication, pour chacune desdites formes visées, d'au moins un instrument de modelage agencé pour être appliqué et maintenu au contact de la région à modeler du nez dudit individu de façon à former un obstacle qui va guider la croissance cartilagineuse voire osseuse du nez.

L'instrument de modelage comporte une surface de contact, qui est au contact du visage, présentant une forme tridimensionnelle stable qui représente ou correspond à ladite forme visée de façon complémentaire. Selon l'invention, il est ainsi prévu une pluralité d'instruments de modelage destinés à être appliqués l'un après l'autre sur le visage dudit individu en vue de guider la croissance cartilagineuse voire osseuse de son nez.

On notera que, dans l'invention, la croissance cartilagineuse est véritablement guidée par la forme de chaque instrument, lequel constitue une sorte de butée que le cartilage en croissance ne peut pas dépasser facilement. La forme du nez qui sera obtenue est donc très proche de celle de l'instrument, et peut ainsi être facilement évaluée et déterminée.

Dans l'art antérieur, même si elle était appliquée, une méthode comme celle du document WO 2011/148309 ne permet pas de proposer une forme déterminée à l'avance pour le rendu final de la forme du nez. De plus, comme les ajustements se font sur les efforts appliqués, et en plus sans en connaître précisément l'intensité, il est très difficile de prévoir le résultat qui sera obtenu. En effet, en modifiant la courbure locale d'un fil ressort, c'est-à-dire en modifiant uniquement et approximativement la force appliquée en quelques points, il est extrêmement difficile de savoir de quelle distance exacte on freinera la croissance de ces quelques points, et plus encore du reste du nez.

On notera que l'invention peut être mise en oeuvre de façon beaucoup plus simple et avec moins de précautions qu'une intervention chirurgicale, présente moins de risques et ne nécessite pas le même type de compétences. Par contre, ce type d'action semble largement insuffisant pour corriger des difformités graves ou fonctionnelles.

Selon une particularité de l'invention, on utilise un moule ou une imprimante 3D pour fabriquer au moins la surface de contact d'au moins un desdits instruments. Dans un mode de réalisation préféré, on utilise un moule ou une imprimante 3D pour fabriquer la surface de contact de chacun des instruments. En outre, le ou les instruments peuvent intégralement être fabriqués par moulage ou par une imprimante 3D. Ces méthodes ont pour avantages d'être simples et peu onéreuses.

De préférence, l'instrument présente une forme stable, c'est-à-dire de sorte que l'instrument soit rigide par rapport à la déformabilité des chairs. Typiquement, la rigidité et/ou l'effort appliqué sont déterminés pour ne pas déformer le cartilage existant. Typiquement, cette rigidité est obtenue avec une élasticité élevée choisie pour suffisamment conserver sa forme géométrique de contact et pour imposer cette forme géométrique de contact de façon répartie et permanente et produisant un effet de moulage à une matière biologique pressant contre l'instrument, ici le cartilage. C'est-à-dire que l'application de l'instrument va produire un effort d'appui là où il est en contact avec le visage, et laisser un espace vide ou de moindre poussée là où la forme de l'instrument est en retrait par rapport à la forme actuelle du visage lorsqu'il est libre. Typiquement, l'action se fait par guidage de la croissance du cartilage vers les espaces vides ou de moindre poussée, et non pas par déformation du cartilage existant.

De manière préférentielle de façons combinables entre elles, le procédé prévoit de :
- relever la forme externe actuelle de la région à modeler du nez de l'individu, fournissant ainsi la forme initiale,
- extrapoler la croissance du nez, à partir de ladite forme initiale relevée, pour élaborer une forme finale dite naturelle représentant la forme que prendra la région à modeler à l'instant final en l'absence d'intervention, et
- déterminer la forme finale visée, à partir de ladite forme finale naturelle et possiblement également de ladite forme initiale.

Le procédé a pour avantage de s'adapter à chaque individu et de prévoir dès le départ la ou les formes futures visées possibles en fonction des paramètres intrinsèques de l'individu. De plus, il est possible de prévoir les différentes formes intermédiaires visées entre la forme initiale et la forme final visée, et donc de prévoir à l'avance la fabrication des instruments de modelage. Il est ainsi possible d'améliorer la prédictibilité de la forme du nez.

Selon une particularité, le procédé prévoit de relever la forme externe de la région à modeler du nez à un instant intermédiaire. Cela permet de corriger éventuellement les prédictions des différentes formes intermédiaires visées en fonction de l'évolution de la croissance cartilagineuse du nez de l'individu, afin d'obtenir la forme finale visée.

Le procédé prévoit plusieurs manières de relever la forme initiale du nez. Il est possible de relever la forme initiale du nez par photographie (par exemple 3D), par radiographie ou par moulage pour obtenir une empreinte de la forme du nez. Il est aussi envisageable d'utiliser une combinaison de tout ou partie de ces méthodes pour obtenir l'empreinte de la forme du nez.

De préférence, l'étape d'extrapolation et/ou de détermination de la forme finale visée comprennent une modification d'image tridimensionnelle informatisée. Cette modification d'image tridimensionnelle peut être réalisée de manière manuelle ou automatisée. Cette caractéristique permet de visualiser les modifications de la forme du nez et permet de définir les formes intermédiaires visées et la forme finale visée.

Selon un deuxième aspect de l'invention, en particulier conforme au premier aspect, il est proposé un procédé de modelage esthétique de la forme externe du nez d'un individu par application sur le visage, et plus particulièrement sur la surface extérieure du nez, d'un instrument de modelage comportant une surface de contact présentant une forme déterminée stable en fonction d'une forme externe future visée pour ledit nez.

Le procédé comprend une réalisation d'une pluralité d'instruments de modelage dont les formes successives sont déterminées pour représenter une succession évolutive d'une ou plusieurs formes intermédiaires visées depuis la forme externe initiale du nez telle que relevée à un état actuel à l'instant initial et jusqu'à la forme externe finale visée du nez. Selon l'invention, le procédé comprend une application desdits instruments sur le visage d'un individu en cours de croissance, c'est-à-dire un individu dont le cartilage du nez est encore en croissance. L'individu est par exemple un enfant ou de préférence un adolescent. L'instant initial correspond un instant suffisamment tôt pour pouvoir agir sur la croissance cartilagineuse. De préférence, l'instant initial correspond sensiblement au point de départ de l'adolescence. L'instant final correspond par exemple à la fin de l'adolescence. Entre l'instant initial et l'instant final, l'application des instruments de modelage a lieu de façon régulière et prolongée, par exemple plusieurs heures par jour, notamment la nuit, ou par semaine, successivement dans le temps et au fur et à mesure de la croissance de l'individu. Les instruments de modelage ont des formes différentes l'un par rapport à l'autre et sont appliqués sur le visage de l'individu l'un après l'autre dans un ordre prédéterminé. Chaque instrument est appliqué sur le nez de l'individu pendant plusieurs heures par jour ou par semaine ; chaque instrument pouvant servir pendant plusieurs semaines ou plusieurs mois. Ce temps est par exemple celui nécessaire afin d'obtenir la forme intermédiaire visée correspondant à l'instrument, avant de poursuivre le procédé de modelage avec un instrument suivant. La forme intermédiaire est par exemple une forme proche voire identique à celle de l'instrument en cours, ou forme dont on estimera que le maintien de l'instrument en cours n'apportera plus d'amélioration, ou un compromis entre les deux. Le procédé de modelage permet ainsi de guider la croissance cartilagineuse de la région à modeler du nez jusqu'à obtenir la forme finale visée ou une forme s'en approchant de manière évolutive grâce à l'application successive des instruments de modelage. De préférence, le modelage du nez s'effectue en guidant la croissance cartilagineuse vers ladite une ou plusieurs formes intermédiaires visées. Cependant, il n'est pas obligatoire de passer par toutes les formes intermédiaires visées. Ainsi, une forme « s'approchant » de la forme finale visée du nez peut être une forme intermédiaire entre la forme finale dite naturelle et la forme finale visée.

Le squelette cartilagineux comporte principalement le cartilage latéral, le cartilage septal et le cartilage alaire. Chaque instrument peut agir sur tout ou partie du squelette cartilagineux afin de guider la croissance cartilagineuse de la région à modeler du nez.

Le procédé de modelage selon l'invention permet d'une part d'améliorer la prédictibilité de la forme du nez obtenu et d'autre part de facilité et d'améliorer la qualité du suivi de l'évolution de la croissance cartilagineuse du nez de l'individu. En effet, une fois les instruments réalisés, il suffit de contrôler la bonne évolution de la croissance cartilagineuse et d'appliquer les instruments successifs dans l'ordre prédéterminé jusqu'à l'instrument final.

Selon une particularité de l'invention, on peut modifier au moins une forme intermédiaire visée en fonction des résultats intermédiaires obtenus aux instants intermédiaires. Cela permet d'adapter ou de corriger les différentes formes intermédiaires visées des instruments de modelage en fonction de l'évolution de la croissance cartilagineuse du nez de l'individu, afin d'obtenir la forme finale visée, ou s'en approchant. Dans certains cas, cela peut conduire à une légère modification de la forme finale visée du nez.

De préférence, on applique une interface siliconée ou en silicone entre l'instrument de modelage et le nez. Cette caractéristique permet d'améliorer le confort d'application de l'instrument sur le nez et de faciliter l'hygiène de la peau et d'éviter des marques temporaires visibles et disgracieuses.

Selon un troisième aspect de l'invention, en particulier conforme au premier et/ou au deuxième aspect, l'invention propose un ensemble d'instruments de modelage à appliquer sur le nez d'un individu en croissance cartilagineuse, un enfant ou un adolescent, pour guider la croissance cartilagineuse du nez. Chaque instrument présente respectivement une surface externe et une surface interne dite surface de contact venant en contact avec au moins une région à modeler dudit nez.

Ledit ensemble comprend au moins :
- un instrument final dont la surface de contact est d'une forme représentant ou correspondant à, de façon complémentaire, une forme finale visée pour le nez dudit individu, et
- un ou plusieurs instruments intermédiaires dont la surface de contact est d'une forme représentant ou correspondant à, de façon complémentaire, une forme intermédiaire entre la forme initiale et la forme finale visée du nez dudit individu.

L'ensemble d'instruments selon l'invention permet d'une part d'améliorer la prédictibilité de la forme du nez obtenu et d'autre part de facilité et d'améliorer la qualité du suivi de l'évolution de la croissance cartilagineuse du nez de l'individu.

De préférence, les instruments sont non réglables, en particulier dans leur forme au repos. De préférence, lors de l'application d'un instrument sur le nez de l'individu, la surface de contact de l'instrument avec la peau du nez correspond à au moins 50% de la surface de la peau du nez. Cela permet de faciliter et d'améliorer la stabilité de l'instrument et donc les formes géométriques de celui-ci et ainsi de s'assurer que l'instrument de modelage permette d'obtenir la forme de nez visée.

Selon un mode de réalisation préféré, les instruments s'étendent le long du dos du nez entre la columelle et la racine du nez. Cette caractéristique permet d'appliquer l'instrument sur toutes les parties cartilagineuses du nez.

De préférence, les instruments sont agencés pour prendre appui sur le front de l'individu. Cette caractéristique permet de positionner l'instrument par rapport au nez. De manière préférentielle, les instruments sont agencés pour prendre appui sur la racine du nez.

De manière préférentielle, les instruments comprennent un dispositif de positionnement et de maintien autour du crâne. Selon un mode de réalisation préféré, le dispositif de positionnement prend au moins un appui élastique sur l'os occipital du crâne. Cela permet de maintenir l'instrument en place par rapport à la tête et de lui fournir tout ou partie de la pression qu'il applique sur le nez. Selon un mode de réalisation préféré, le dispositif de positionnement élastique prend la forme de sangles ou de bandeaux élastiques.

Optionnellement, les instruments sont fabriqués en résine, par exemple en matière durcissable, synthétique ou végétale.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
- la figure 1 illustre un procédé de modelage esthétique de la forme externe du nez, selon l'invention, par des vues de profil d'un visage entre une forme initiale et une forme finale naturelle d'une part et une forme finale visée obtenue via au moins deux instruments de modelage d'autre part ;
- la figure 2 correspond au processus b de la figure 1 et illustre l'effet de l'application des instruments de modelage sur la forme du nez, lors de la croissance de l'individu ;
- la figure 3a est une vue de droite d'un instrument de modelage, selon l'invention ;
- la figure 3b est une vue de face d'un instrument de modelage, selon l'invention ;
- la figure 4 est une vue de profil d'un visage, sur lequel est placé un instrument de modelage du nez, l'instrument étant transparent ;
- la figure 5 est une image tridimensionnelle informatisée d'un visage montrant notamment le nez dans sa forme initiale ;
- la figure 6 est une image tridimensionnelle informatisée du visage représenté en figure 5 montrant le nez dans une forme intermédiaire.

Ces modes de réalisation n'étant nullement limitatifs, on pourra notamment considérer des variantes de l'invention ne comprenant qu'une sélection de caractéristiques décrites par la suite isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

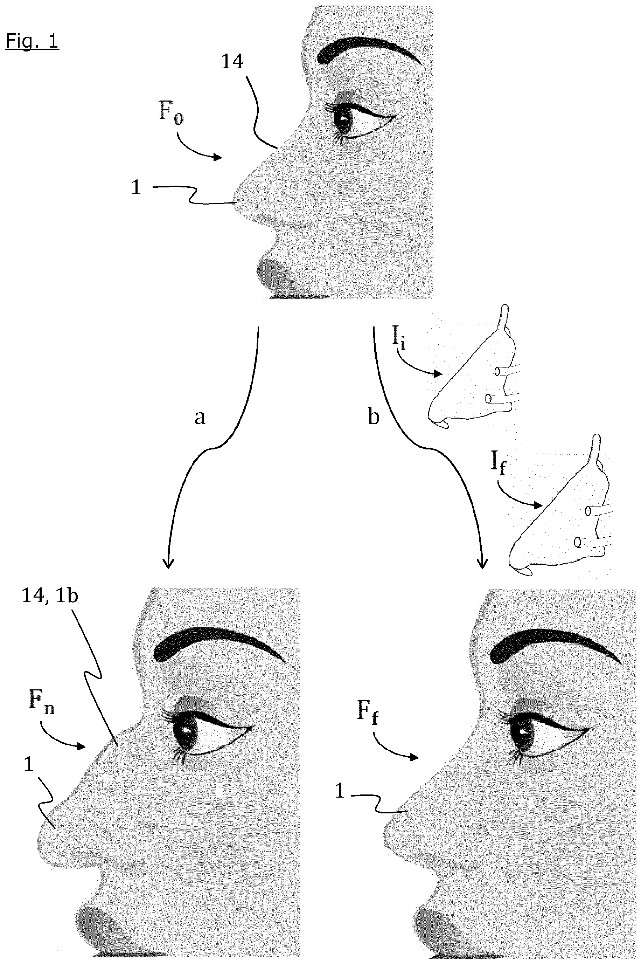

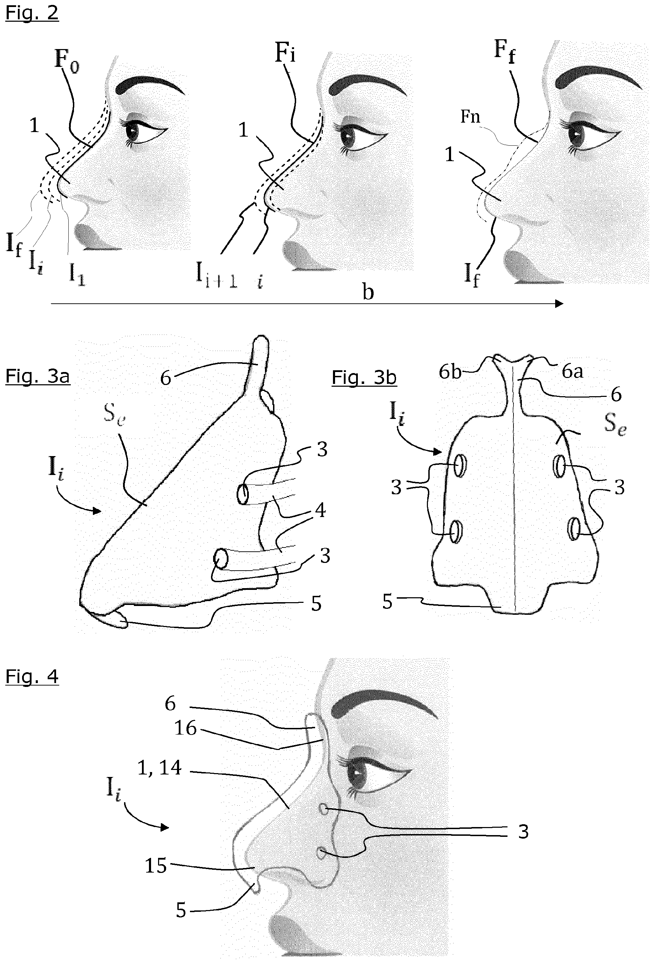

On va tout d'abord décrire, en référence aux figures 1 à 4, un procédé de modelage esthétique de la forme externe d'un nez 1 d'un individu. Selon l'invention, le procédé consiste à appliquer sur le visage d'un individu en croissance et plus particulièrement sur le nez de celui-ci, au moins deux instrument de modelage Iᵢ et I_{f} (flèche b sur les figures 1 et 2) afin de guider la croissance cartilagineuse du nez et ainsi d'obtenir une forme externe finale du nez visée F_{f}, à la fin de la croissance de l'individu, dit instant t_{f}, et qui est différente d'une forme externe naturelle Fₙ qui serait obtenue naturellement en l'absence d'intervention (flèche a sur la figure 1).

L'individu en cours de croissance est un individu dont la croissance cartilagineuse du nez est en cours, c'est-à-dire en général un enfant ou un adolescent. En référence aux figures 1 et 2, il est fréquent que l'individu lors de l'enfance ou la pré-adolescence, dit instant t₀, présente un nez dont la forme externe, dite forme initiale F₀, donne l'impression d'une harmonie visuelle. En revanche, lors de l'adolescence, la croissance cartilagineuse du nez peut faire apparaître une bosse 1b sur le dos 14 du nez, typiquement du fait de la croissance excessive du cartilage latéral et/ou du cartilage septal. Cette bosse est particulièrement visible de profil et est définitive. Une fois la croissance de l'individu terminée, le nez présente une forme externe finale dite naturelle Fₙ mais qui ne donne plus satisfaction quant à l'impression d'harmonie visuelle qu'elle donne.

On entend par instrument de modelage Iᵢ, une pièce telle une attelle ou un masque recouvrant au moins la surface extérieure d'une région du nez, dite « région à modeler ». En particulier, l'instrument de modelage Iᵢ comporte une surface interne de contact Sᵢ présentant une forme déterminée stable et prend appui sur la région à modeler du nez (figures 2 et 4). La figure 2 montre une ligne de contact représentant l'évolution de la surface de contact au cours des différents instruments. Les différents contours représentés correspondent aux différents instruments de modelage (Iᵢ, Iᵢ, Iᵢ₊₁, I_{f}) appliqués sur le dos du nez, celui en cours étant en trait plein et les autres en pointillés.

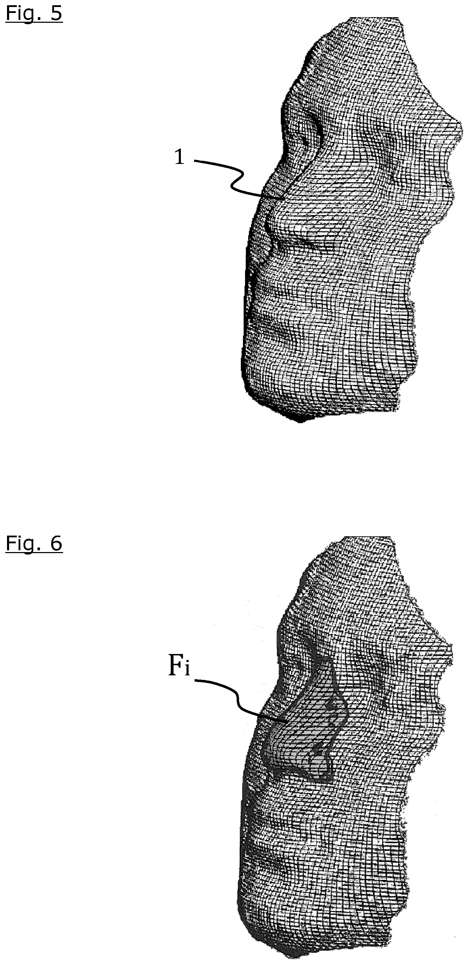

Avant d'appliquer les instruments de modelage Iᵢ, le procédé de modelage prévoit, à l'instant t₀ de relever la forme externe actuelle de la région à modeler du nez de l'individu afin d'obtenir la forme initiale F₀ du nez (figure 1), par exemple selon des méthodes connues (voir figure 5, décrite ci-dessous). Ensuite, à partir de cette forme initiale F₀, on extrapole la croissance cartilagineuse du nez pour élaborer la forme finale dite naturelle Fₙ, représentant la forme que prendra la région à modeler du nez en l'absence d'intervention. On détermine la forme future visée F_{f} en fonction de la forme finale dite naturelle Fₙ, de la forme initiale F₀ et de l'appréciation subjective de l'individu qui souhaite embellir la forme externe de son nez. De préférence, la forme finale visée du nez est déterminée de manière à trouver un compromis entre la forme finale dite naturelle prévisible et la forme finale que souhaite l'individu afin que la forme finale visée soit plus aisément atteignable. En outre on détermine au moins une forme externe intermédiaire visée Fᵢ, représentant une forme que l'on cherche à obtenir (ou du moins à approcher, ou évoluer vers) en un instant intermédiaire tᵢ situé entre l'instant initial t₀ et l'instant final t_{f}. Cela permet de modeler la région du nez de manière progressive. Enfin, on fabrique les instruments de modelage intermédiaires Iᵢ et l'instrument de modelage final I_{f} comportant chacun une surface interne de contact Sᵢ ou S_{f} correspondant de façon complémentaire aux formes intermédiaires Fᵢ ou à la forme future visée F_{f}. On peut fabriquer les instruments de modelage simultanément ou au fur et à mesure de l'évolution de la croissance de l'individu.

Selon l'invention, le procédé de modelage prévoit au moins un instrument de modelage intermédiaire Iᵢ et un instrument de modelage final I_{f}. Chacun des instruments de modelage intermédiaires Iᵢ comportent une surface de contact Sᵢ présentant une forme déterminée stable en fonction de la forme externe future visé F_{f} donnant l'impression d'une harmonie visuelle afin d'éviter la forme externe finale dite naturelle Fₙ à la fin de la croissance de l'individu. L'instrument de modelage final I_{f} comporte une surface de contact S_{f} correspondant à la forme externe future visée F_{f}.

Selon l'invention, et en référence à la figure 2, on applique une succession d'instruments de modelage Iᵢ dont les formes successives sont déterminées pour représenter une succession évolutive de une ou plusieurs formes intermédiaires Fᵢ visées depuis la forme externe initiale F₀ du nez telle que relevée à un état actuel (pendant l'enfance ou l'adolescence) à l'instant initial t₀ et jusqu'à la forme externe finale visée F_{f} du nez à la fin de la croissance. La succession d'instruments permet de guider la croissance cartilagineuse du nez de façon progressive. La figure 2, en particulier la vue à gauche, présente une superposition de lignes de contact correspondant aux instruments de modelage, en particulier l'instrument de modelage I₁ au début du procédé de modelage, puis au-dessus un instrument de modelage intermédiaire Iᵢ et encore au-dessus un instrument de modelage final I_{f} correspondant au dernier instrument appliqué pour obtenir la forme finale visée F_{f}, montrant différents instruments de modelage qui vont être appliqués sur la région à modeler du nez. Il est bien clair que le nombre d'instruments successifs peut varier, par exemple en fonction des individus ou des procédures mis en œuvre.

En référence aux figures 2 et 4, les instruments de modelage Iᵢ doivent être appliqués sur le visage de l'individu en cours de croissance de façon régulière et prolongée comme les dispositifs orthodontiques. Il est préférable d'appliquer les instruments plusieurs heures par jour, notamment la nuit, ou par semaine de façon à former un obstacle à la croissance cartilagineuse du nez et ainsi de la guider. Chaque instrument de modelage Iᵢ constitue une sorte de butée que le cartilage en croissance ne peut pas dépasser facilement. La figure 2, en particulier la vue du milieu, illustre l'effet, vu de profil, des instruments intermédiaires Iᵢ et Iᵢ₊₁ sur le dos 14 du nez. La forme du nez qui sera obtenue est donc très proche de celle de l'instrument, et peut ainsi être facilement évaluée et déterminée. Bien sûr l'instrument de modelage n'a pas pour but d'empêcher la croissance cartilagineuse, mais de la guider dans la forme de l'instrument. Une fois la forme intermédiaire Fᵢ du nez obtenue, ou proche, on applique l'instrument de modelage suivant Iᵢ₊₁ et ainsi de suite (suggéré par la ligne en pointillée sur la figure 2). Les instruments de modelage Iᵢ sont appliqués successivement dans le temps, par exemple tous les trois mois, et au fur et à mesure de la croissance de l'individu permettant ainsi de guider la croissance cartilagineuse de la région à modeler du nez jusqu'à obtenir la forme finale visée F_{f} ou s'y approchant grâce au dernier instrument de modelage I_{f}. Le procédé de modelage ne prévoit pas un nombre limité d'instruments intermédiaires.

Afin de rendre l'application de l'instrument plus confortable et d'éviter des difficultés d'hygiène de la peau, on applique une interface siliconée entre l'instrument de modelage et le nez.

En fonction des résultats intermédiaires obtenus aux instants intermédiaires, par exemple en cas de décalage entre les formes intermédiaires visées des instruments et la forme externe réelle du nez, il est possible de modifier la forme externe future finale visée et/ou les formes intermédiaires visées. On peut alors fabriquer de nouveaux instruments de modelage intermédiaires en remplacement des instruments intermédiaires prévus initialement.

En référence aux figures 2 à 4, on va maintenant décrire un ensemble d'instruments de modelage à appliquer sur le nez 1 d'un individu en croissance cartilagineuse pour guider la croissance cartilagineuse du nez. Chaque instrument de modelage Iᵢ présente une surface externe Sₑ et une surface interne dite surface de contact Sᵢ venant en contact avec au moins une région à modeler du nez. Comme développé ci-dessus l'ensemble d'instruments comprend :
- un instrument final I_{f} dont la surface de contact est d'une forme représentant ou correspondant à, de façon complémentaire, une forme finale visée F_{f} pour le nez dudit individu, et
- un ou plusieurs instruments intermédiaires Iᵢ dont la surface de contact Sᵢ est d'une forme représentant ou correspondant à, de façon complémentaire, une forme intermédiaire Fᵢ entre la forme initiale F₀ et la forme finale visée F_{f} du nez 1 dudit individu (figure 2).

Les instruments de modelage Iᵢ, I_{f} se différencient les uns des autres au moins par une surface interne de contact de forme différente pour permettre de donner une forme déterminée au nez après application successive des différents instruments dans le temps et au fur et à mesure de la croissance de l'individu. La figure 2 montre en particulier une ligne de contact, correspondant à chaque instrument de modelage (Iᵢ, Iᵢ, Iᵢ₊₁, I_{f}) appliqué sur le dos du nez.

En référence aux figures 3a, 3b et 4, les instruments de modelage Iᵢ s'étendent le long du dos du nez entre la columelle 15 du nez et la racine 16 du nez afin de pouvoir se placer sur la surface extérieur du nez, et plus particulièrement au-dessus des parties cartilagineuses du nez. Par exemple, l'instrument peut s'appuyer sur le dos 14 du nez, et sur la columelle 15 du nez par une extrémité 5 de l'instrument permettant d'exercer une contention vers le haut.

En outre, l'instrument comporte une extrémité supérieure 6 pour prendre appui sur le front de l'individu et permettre de positionner l'instrument de modelage par rapport au nez mais sans agir sur le massif facial. De manière préférentielle, et en référence à la figure 3b, l'extrémité supérieure 6 comprend deux lobes 6a, 6b prévus pour prendre appui de part et d'autre de l'os du nez.

De préférence, l'instrument de modelage comprend un dispositif de positionnement élastique 4 autour du crâne qui prennent appui sur l'os occipital du crâne afin de maintenir l'instrument de modelage Iᵢ avec une pression suffisante sur le nez de l'individu de manière à ce que l'instrument forme butée pour la croissance cartilagineuse. En référence à la figure 3a, le dispositif de positionnement présente des élastiques ou des bandeaux élastiques 4 qui sont fixés à la surface externe Sₑ de l'instrument de modelage et entourent le crâne de l'individu. Selon un mode de réalisation particulier (non représenté), l'instrument comprend un taquet occipital agencé pour se placer par conformation de forme près de l'occiput du crâne et pour faire passer les élastiques par le taquet.

En référence aux figures 3a, 3b et 4, les instruments de modelage comprennent des systèmes de fixation 3 sur la surface externe Sₑ de l'instrument pour accrocher le dispositif de positionnement élastique. Selon un premier exemple de mode de réalisation, les systèmes de fixation 3 comprennent des plots de forme cylindrique autour desquels les bandeaux élastiques peuvent s'accrocher. Selon un second exemple de mode de réalisation, l'enveloppe de l'instrument présente des perçages pour y attacher les bandeaux élastiques.

On va maintenant décrire en référence aux figures 5 et 6, un procédé de réalisation d'instruments de modelage de la forme du nez 1 d'un individu. Comme on l'a vu précédemment avec le procédé de modelage, il est nécessaire d'appliquer au moins un instrument de modelage intermédiaire Iᵢ pour modeler la région du nez en croissance cartilagineuse afin d'obtenir des formes intermédiaires visées Fᵢ et un instrument de modelage finale I_{f} peu de temps avant la fin de la croissance ou lors de la fin de la croissance, pour obtenir la forme externe finale F_{f} du nez. Tout d'abord et en référence à la figure 5, il est nécessaire de relever la forme externe actuelle de la région à modeler du nez 1 de l'individu à un instant dit initial t₀ afin d'obtenir la forme initiale F₀. Plusieurs méthodes sont possibles pour relever la forme initiale F₀ du nez. Par exemple, on photographie le visage en utilisant le procédé dit « photographie 3D ». On peut aussi utiliser la radiographie 3D. Il est aussi prévu de mouler le visage afin d'obtenir une empreinte physique. Enfin, il est prévu d'utiliser une combinaison de tout ou partie de ces méthodes. Selon un mode de réalisation préféré, le visage de l'individu et plus particulièrement la forme initiale du nez sont numérisés afin d'obtenir une image tridimensionnelle informatisée du nez visible, sur un ordinateur (figure 5). On connaît par exemple du document US2006/0097422 A1 un procédé permettant d'obtenir une image tridimensionnelle d'une surface d'une partie d'un corps, de manipuler ladite image afin d'obtenir une forme ou profil désiré.

Par exemple de façon similaire au document US2006/0097422 A, à partir de l'image informatisée de la forme initiale du nez, on manipule cette image pour extrapoler la croissance cartilagineuse du nez, via un logiciel de traitement d'images. L'image tridimensionnelle initiale est modifiée afin d'obtenir une image tridimensionnelle de la forme finale dite naturelle. Il est ainsi possible de montrer l'évolution de la croissance du nez et la forme finale dite naturelle Fₙ représentant la forme que prendrait le nez ou la région à modeler du nez après la fin de la croissance de l'individu et en l'absence d'intervention.

En fonction de la forme initiale et de la forme finale naturelle évaluée, on extrapole ou détermine la forme finale visée F_{f}, via un logiciel de traitement d'images, en modifiant de manière manuelle ou automatisée l'image tridimensionnelle informatisée initiale. Une fois que la forme finale visée est définie, on détermine informatiquement au moins une forme intermédiaire Fᵢ du nez représentant une forme que l'on cherche à obtenir en un instant intermédiaire situé entre l'instant initial et l'instant final de manière à guider progressivement la croissance cartilagineuse du nez entre la forme initiale et la forme finale visée. La figure 6 montre un exemple de forme intermédiaire du nez. La surface grisée est la surface extérieure du nez et correspond à une forme intermédiaire visée Fᵢ que l'on cherche à obtenir à un instant intermédiaire. La détermination de cette surface permet aussi de définir la surface interne dit surface de contact Sᵢ de l'instrument de modelage puisque la surface de contact de l'instrument intermédiaire est complémentaire de la surface externe du nez à l'instant intermédiaire considéré.

On fabrique, pour chacune desdites formes visées, au moins un instrument de modelage Iᵢ agencé pour être appliqué et maintenu au contact de la région à modeler du nez dudit individu afin de guider la croissance cartilagineuse du nez dudit individu. Les instruments de modelage Iᵢ sont réalisés de sorte qu'ils comportent une surface de contact Sᵢ présentant une forme stable, c'est-à-dire qu'ils présentent une rigidité suffisante pour que chaque instrument représente ou corresponde à ladite forme visée de façon complémentaire, et garde suffisamment cette forme une fois appliqué pour agir sur le nez.

Il peut ainsi être prévu autant d'instruments de modelage que nécessaire pour les appliquer l'un après l'autre sur le visage dudit individu en vue de guider la croissance cartilagineuse de son nez.

Bien sûr, l'invention n'est pas limitée aux exemples qui viennent d'être décrits et de nombreux aménagements peuvent être apportés à ces exemples sans sortir du cadre de l'invention.

## Revendications

1. Procédé de réalisation d'instruments de modelage de la forme du nez (1) d'un individu, ledit procédé comprenant
- à partir d'une forme initiale (F₀), représentant la forme initiale d'une surface extérieure d'une région du nez dudit individu, dite région à modeler, telle qu'elle existe à un instant initial (t₀) :
∘ détermination d'une forme finale visée (F_{f}), représentant la forme que l'on cherche à obtenir en un instant final (t_{f}), pour ladite région à modeler du nez dudit individu,
∘ détermination d'au moins une forme intermédiaire visée (Fᵢ), représentant une forme que l'on cherche à obtenir en un instant intermédiaire (tᵢ) situé entre l'instant initial (t₀) et l'instant final (t_{f}), pour ladite région à modeler du nez dudit individu ; et
- fabrication, pour chacune desdites formes visées, d'au moins un instrument de modelage (Iᵢ) agencé pour être appliqué et maintenu au contact de la région à modeler du nez dudit individu de façon à former un obstacle à la croissance cartilagineuse du nez dudit individu,
ledit instrument de modelage comportant une surface de contact (Sᵢ) présentant une forme stable qui représente ou correspond de façon complémentaire à ladite forme visée, formant ainsi une pluralité d'instruments de modelage prévus pour être appliqués l'un après l'autre sur le visage dudit individu en vue de guider la croissance cartilagineuse de son nez,
procédé au cours duquel :
- on relève la forme externe actuelle de la région à modeler du nez (1) de l'individu, fournissant ainsi la forme initiale (F₀),
**caractérisé en ce que** :
- à partir de ladite forme initiale (F₀), on extrapole la croissance du nez pour élaborer une forme finale dite naturelle (Fₙ), représentant la forme que prendra la région à modeler à l'instant final en l'absence d'intervention, et
- à partir de ladite forme finale naturelle (et possiblement de ladite forme initiale), on détermine la forme finale visée (F_{f}).

2. Procédé de réalisation d'instruments selon la revendication 1, **caractérisé en ce qu'**on utilise un moule ou une imprimante 3D pour fabriquer au moins la surface de contact (Sᵢ) d'au moins un desdits instruments (Iᵢ).

3. Procédé de réalisation d'instruments selon la revendication 1, **caractérisé en ce qu'**on relève la forme initiale du nez par photographie (par exemple 3D) ou radiographie (par exemple 3D) ou par moulage ou une combinaison de tout ou partie de ces méthodes.

4. Procédé de réalisation d'instruments selon l'une quelconque des revendications 1 à 3, **caractérisé en ce que** l'étape d'extrapolation et/ou de détermination de la forme finale visée (F_{f}) comprennent une modification d'image tridimensionnelle informatisée.

5. Procédé de modelage esthétique de la forme externe du nez (1) d'un individu par application sur le visage d'un instrument de modelage comportant une surface de contact présentant une forme déterminée stable en fonction d'une forme externe future visée pour ledit nez (1),
**caractérisé en ce qu'il** comprend une réalisation d'une pluralité d'instruments (Iᵢ) de modelage dont les formes successives sont déterminées pour représenter une succession évolutive de une ou plusieurs formes intermédiaires (Fᵢ) visées depuis la forme externe initiale (F₀) du nez telle que relevée à un état actuel à l'instant initial (t₀) et jusqu'à la forme externe finale visée (F_{f}) du nez, selon l'une des revendications 1 à 4 ; et
**en ce qu'**il comprend une application desdits instruments (Iᵢ) sur le visage d'un individu en cours de croissance, de façon régulière et prolongée, successivement dans le temps et au fur et à mesure de la croissance de l'individu, permettant ainsi de guider la croissance cartilagineuse de la région à modeler jusqu'à obtenir la forme finale visée (F_{f}) ou une forme s'en approchant.

6. Procédé de modelage selon la revendication 5, caractérisé en que l'on modifie au moins une forme intermédiaire (Fᵢ₊₁) du nez (1) en fonction des résultats intermédiaires obtenus aux instants intermédiaires (tᵢ).

7. Procédé de modelage selon la revendication 5 ou 6, **caractérisé en ce qu'**on applique une interface siliconée ou en silicone entre l'instrument de modelage et le nez (1).

8. Ensemble d'instruments de modelage à appliquer sur le nez (1) d'un individu en croissance cartilagineuse pour guider la croissance cartilagineuse du nez, réalisé selon un procédé selon l'une quelconque des revendications 1 à 4,
chaque instrument (Iᵢ) présentant respectivement une surface externe et une surface interne (Sᵢ) dite surface de contact venant en contact avec au moins une région à modeler dudit nez,
ledit ensemble comprenant au moins
- un instrument final (I_{f}) dont la surface de contact est d'une forme représentant ou correspondant à, de façon complémentaire, une forme finale visée (F_{f}) pour le nez dudit individu, et
- un ou plusieurs instruments intermédiaires (Iᵢ) dont la surface de contact est d'une forme représentant ou correspondant à, de façon complémentaire, une forme intermédiaire (Fᵢ) entre la forme initiale (F₀) et la forme finale visée (F_{f}) du nez (1) dudit individu.

9. Ensemble d'instruments de modelage à appliquer sur le nez, selon la revendication 8, **caractérisé en ce que** les instruments (Iᵢ) sont non réglables.

10. Ensemble d'instruments de modelage à appliquer sur le nez selon la revendication 8 ou 9, **caractérisé en ce que** les surfaces de contact des instruments (Iᵢ) sont agencées pour s'étendre le long du dos du nez entre la columelle (15) et la racine (16) du nez.

11. Ensemble d'instruments de modelage à appliquer sur le nez selon l'une des revendications 8 à 10, **caractérisé en ce que** les instruments (Iᵢ) comprennent une extrémité supérieure (6) pour prendre appui sur le front de l'individu.

12. Ensemble d'instruments de modelage à appliquer sur le nez selon l'une des revendications 8 à 11, **caractérisé en ce que** les instruments (Iᵢ) comprennent un dispositif de positionnement élastique (4) autour du crâne qui prennent au moins appui sur l'os occipital du crâne.

13. Ensemble d'instruments de modelage à appliquer sur le nez selon l'une des revendications 8 à 12, **caractérisé en ce que** les instruments (Iᵢ) sont fabriqués en résine.

## Patentansprüche

1. Verfahren zur Ausführung von Modellierungsinstrumenten für die Form der Nase (1) eines Menschen, umfassend
- anhand einer Ausgangsform (F₀), die die Ausgangsform einer Außenoberfläche eines sogenannten zu modellierenden Bereichs der Nase des Menschen so darstellt, wie dieser Bereich zu einem Ausgangszeitpunkt (t₀) besteht:
∘ - Bestimmung einer anvisierten Endform (F_{f}), die die zu einem Endzeitpunkt (t_{f}) zu erzielende Form für den zu modellierenden Bereich der Nase des Menschen darstellt,
∘ - Bestimmung mindestens einer anvisierten Zwischenform (Fᵢ), die für den zu modellierenden Bereich der Nase des Menschen eine Form darstellt, welche zu einem Zwischenzeitpunkt (tᵢ) erzielt werden soll, welcher zwischen dem Ausgangszeitpunkt (t₀) und dem Endzeitpunkt (t_{f}) liegt; und
- für jede der anvisierten Formen Herstellung mindestens eines Modellierungsinstruments (Iᵢ), welches dazu angeordnet ist, aufgebracht und im Kontakt mit dem zu modellierenden Bereich der Nase des Menschen derart gehalten zu werden, um ein Hindernis zum Knorpelwachstum der Nase des Menschen zu bilden,
wobei das Modellierungsinstrument eine Kontaktoberfläche (Sᵢ) umfasst, welche eine stabile Form aufweist, die die anvisierte Form darstellt oder ihr ergänzend entspricht, wobei somit eine Vielzahl von Modellierungsinstrumenten gebildet wird, die dazu vorgesehen sind, nacheinander auf das Gesicht des Menschen aufgebracht zu werden, um das Knorpelwachstum dessen Nase zu lenken,
Verfahren bei welchem:
- die aktuelle äußere Form des zu modellierenden Bereichs der Nase (1) des Menschen erfasst und somit die Ausgangsform (F₀) bereitgestellt wird,
**dadurch gekennzeichnet, dass**:
- anhand der Ausgangsform (F₀) das Wachstum der Nase zur Erstellung einer sogenannten natürlichen Endform (Fₙ) hochgerechnet wird, welche die Form darstellt, die der zu modellierende Bereich zum Endzeitpunkt bei nicht stattfindendem Eingriff annehmen wird, und
- anhand der natürlichen Endform (und möglicherweise der Ausgangsform) wird die anvisierte Endform (F_{f}) bestimmt.

2. Verfahren zur Ausführung von Instrumenten nach Anspruch 1,
**dadurch gekennzeichnet, dass** eine Form oder ein 3D-Drucker zur Herstellung mindestens der Kontaktoberfläche (Sᵢ) mindestens eines der Instrumenten (Iᵢ) verwendet wird.

3. Verfahren zur Ausführung von Instrumenten nach Anspruch 1,
**dadurch gekennzeichnet, dass** die Ausgangsform der Nase mittels Lichtbildaufnahme (zum Beispiel 3D) oder einer Röntgenaufnahme (zum Beispiel 3D) oder mittels Formen oder einer Mischung aus allen oder einigen dieser Verfahrensmethoden erfasst wird.

4. Verfahren zur Ausführung von Instrumenten nach einem der Ansprüche 1 bis 3, **dadurch gekennzeichnet, dass** der Schritt der Hochrechnung und/oder der Bestimmung der anvisierten Endform (F_{f}) eine Änderung eines rechnergestützten dreidimensionalen Bildes umfassen.

5. Verfahren zur ästhetischen Modellierung der äußeren Form der Nase (1) eines Menschen durch Aufbringen eines Modellierungsinstruments auf das Gesicht, welches Modellierungsinstrument eine Kontaktoberfläche enthält, die eine in Abhängigkeit von einer für die Nase (1) anvisierten äußeren, künftigen Form stabil bestimmte Form aufweist,
**dadurch gekennzeichnet, dass** es eine Ausführung einer Vielzahl von Modellierungsinstrumenten (Iᵢ) umfasst, deren aufeinander folgende Formen dazu bestimmt sind, eine entwicklungstechnische Reihenfolge einer oder mehrerer anvisierten Zwischenformen (Fᵢ) darzustellen, von der äußeren Ausgangsform (F₀) der Nase ausgehend, so wie sie in einem aktuellen Zustand zum Ausgangszeitpunkt (t₀) erfasst wird und bis zu der anvisierten äußeren Endform (F_{f}) der Nase, nach einem der Ansprüche 1 bis 4; und dadurch, dass es ein regelmäßiges und langfristiges , zeitlich aufeinander folgendes Aufbringen der Instrumenten (Iᵢ) auf das Gesicht eines sich im Wachstum befindlichen Menschen je nach Wachstum des Menschen umfasst, wodurch das Knorpelwachstum des zu modellierenden Bereichs geleitet wird, bis die anvisierte Endform (F_{f}) oder eine sich annähernde Form erzielt wird.

6. Modellierungsverfahren nach Anspruch 5, **dadurch gekennzeichnet, dass** mindestens eine Zwischenform (Fᵢ₊₁) der Nase (1) in Abhängigkeit von den zu den Zwischenzeitpunkten (tᵢ) erzielten Zwischenergebnissen geändert wird.

7. Anbringungsverfahren nach Anspruch 5 oder 6, **dadurch gekennzeichnet, dass** eine silikongestütze Trennfläche oder Silikontrennfläche zwischen dem Modellierungsinstrument und der Nase (1) aufgebracht wird.

8. Anordnung von Modellierungsinstrumenten, die auf die Nase (1) eines sich im Knorpelwachstum befindlichen Menschen zur Leitung des Knorpelwachstums der Nase aufzubringen ist, welche Anordnung nach einem Verfahren nach einem der Ansprüche 1 bis 4 ausgeführt wird,
wobei jedes Instrument (Iᵢ) jeweils eine äußere Oberfläche und eine innere, sogenannte Kontaktoberfläche (Sᵢ) aufweist, die mit mindestens einem zu modellierenden Bereich der Nase in Kontakt gebracht wird,
mindestens umfassend
- ein Endinstrument (I_{f}), dessen Kontaktoberfläche eine Form aufweist, die eine für die Nase des Menschen anvisierte Endform (F_{f}) darstellt oder ihr ergänzenderweise entspricht, und
- ein oder mehrere Zwischeninstrumente (Iᵢ), deren Kontaktoberfläche eine Form aufweist, die eine Zwischenform (Fᵢ) zwischen der Ausgangsform (F₀) und der anvisierten Endform (F_{f}) der Nase (1) des Menschen darstellt oder ihr ergänzenderweise entspricht.

9. Anordnung von auf die Nase aufzubringenden Modellierungsinstrumenten nach Anspruch 8, **dadurch gekennzeichnet, dass** die Instrumente (Iᵢ) nicht einstellbar sind.

10. Anordnung von auf die Nase aufzubringenden Modellierungsinstrumenten nach Anspruch 8 oder 9, **dadurch gekennzeichnet, dass** die Kontaktoberflächen der Instrumente (Ii) derart angeordnet sind, sodass sie sich entlang des Nasenrückens zwischen dem Nasensteg (15) und der Nasenwurzel (16) erstrecken.

11. Anordnung von auf die Nase aufzubringenden Modellierungsinstrumenten nach einem der Ansprüche 8 bis 10, **dadurch gekennzeichnet, dass** die Instrumente (Iᵢ) ein oberes Ende (6) umfassen, um sich auf der Stirn des Menschen abzustützen.

12. Anordnung von auf die Nase aufzubringenden Modellierungsinstrumenten nach einem der Ansprüche 8 bis 11, **dadurch gekennzeichnet, dass** die Instrumente (Iᵢ) eine Vorrichtung zur elastischen Lagerung (4) um den Schädel herum umfassen, die sich wenigstens auf dem Hinterhauptbein des Schädels abstützen.

13. Anordnung von auf die Nase aufzubringenden Modellierungsinstrumenten nach einem der Ansprüche 8 bis 12, **dadurch gekennzeichnet, dass** die Instrumente (Iᵢ) aus Harz hergestellt sind.

## Claims

1. Method for producing instruments for modelling the shape of the nose (1) of an individual, said method comprising:
- based on an initial shape (F₀), representing the initial shape of an external surface of a region of the nose of said individual, called modelling region, as it exists at an initial time (t₀):
∘ determining a target final shape (F_{f}), representing the shape that it is sought to obtain at a final time (t_{f}), for said modelling region of the nose of said individual,
∘ determining at least one target intermediate shape (Fᵢ), representing a shape that it is sought to obtain at an intermediate time (tᵢ) situated between the initial time (t₀) and the final time (t_{f}), for said modelling region of the nose of said individual, and
- producing, for each of said target shapes, at least one modelling instrument (Iᵢ) arranged in order to be applied and held in contact with the modelling region of the nose of said individual, in such a way as to form an obstacle to the cartilaginous growth of the nose of said individual,
said modelling instrument including a contact surface (Sᵢ) having a stable shape that represents or corresponds complementarily to said target shape, thus forming a plurality of modelling instruments provided in order to be applied one after another on the face of said individual with a view to guiding the cartilaginous growth of their nose,
method during which:
- the current external shape of the modelling region of the nose (1) of the individual is recorded, thus providing the initial shape (F₀),
**characterized in that**:
- based on said initial shape (F₀), the growth of the nose is extrapolated in order to elaborate a final shape, called natural shape (Fₙ), representing the shape that will be adopted by the modelling region at the final time in the absence of intervention, and
- based on said natural final shape (and possibly said initial shape) the target final shape (F_{f}) is determined.

2. Method for producing instruments according to claim 1, **characterized in that** a mould or a 3D printer is used in order to produce at least the contact surface (Sᵢ) of at least one of said instruments (Iᵢ).

3. Method for producing instruments according to claim 1, **characterized in that** the initial shape of the nose is recorded by photography (for example 3D) or radiography (for example 3D) or by moulding or a combination of all or some of these methods.

4. Method for producing instruments according to any one of claims 1 to 3, **characterized in that** the step of extrapolation and/or determination of the target final shape (F_{f}) comprises modifying a computerized three-dimensional image.

5. Method for aesthetic modelling of the external shape of the nose (1) of an individual by applying on the face a modelling instrument including a contact surface having a stable determined shape depending on a target future external shape for said nose (1),
**characterized in that** it comprises producing a plurality of modelling instruments (Iᵢ), the successive shapes of which are determined in order to represent a progressive succession of one or more target intermediate shapes (Fᵢ) from the initial external shape (F₀) of the nose as recorded in a current state at the initial time (t₀) up to the target final external shape (F_{f}) of the nose, according to one of claims 1 to 4, and
**in that** it comprises a regular and prolonged application of said instruments (Iᵢ) on the face of an individual who is growing, successively over time, and as the individual grows, thus making it possible to guide the cartilaginous growth of the modelling region until the target final shape (F_{f}) is obtained, or a shape close thereto.

6. Modelling method according to claim 5, **characterized in that** at least one intermediate shape (Fᵢ₊₁) of the nose (1) is modified depending on the intermediate results obtained at the intermediate times (tᵢ).

7. Modelling method according to claim 5 or 6, **characterized in that** a siliconized or silicone interface is applied between the modelling instrument and the nose (1).

8. Set of modelling instruments to be applied on the nose (1) of an individual whose cartilage is growing, in order to guide the cartilaginous growth of the nose, carried out according to a method according to any one of claims 1 to 4,
each instrument (Iᵢ) having respectively an outer surface and an inner surface (Sᵢ) called contact surface coming into contact with at least one modelling region of said nose,
said set comprising at least:
- a final instrument (I_{f}), the contact surface of which has a shape representing or corresponding complementarily to a target final shape (F_{f}) for the nose of said individual, and
- one or more intermediate instruments (Iᵢ), the contact surface of which has a shape representing or corresponding complementarily to an intermediate shape (Fᵢ) between the initial shape (F₀) and the target final shape (F_{f}) for the nose (1) of said individual.

9. Set of modelling instruments to be applied on the nose according to claim 8, **characterized in that** the instruments (Iᵢ) are not adjustable.

10. Set of modelling instruments to be applied on the nose according to claim 8 or 9, **characterized in that** the contact surfaces of the instruments (Iᵢ) are arranged in order to extend along the bridge of the nose between the columella (15) and the nasal root (16).

11. Set of modelling instruments to be applied on the nose according to one of claims 8 to 10, **characterized in that** the instruments (Iᵢ) comprise an upper end (6) for bearing on the forehead of the individual.

12. Set of modelling instruments to be applied on the nose according to one of claims 8 to 11, **characterized in that** the instruments (Iᵢ) comprise an elastic positioning device (4) around the cranium which bear at least on the occipital bone of the cranium.

13. Set of modelling instruments to be applied on the nose according to one of claims 8 to 12, **characterized in that** the instruments (Iᵢ) are produced from resin.
